Europäisches Patentamt

European Patent Office

Office européen des brevets

(19)

(11) Veröffentlichungsnummer : **0 053 257**
**B1**

(12) **EUROPÄISCHE PATENTSCHRIFT**

(45) Veröffentlichungstag der Patentschrift :
25.07.84

(51) Int. Cl.³ : **C 07 H 13/10**

(21) Anmeldenummer : **81108459.9**

(22) Anmeldetag : **17.10.81**

(54) **Verfahren zur Herstellung eines beta-D-Glucopyranosederivates.**

(30) Priorität : **27.11.80 CH 8808/80**

(43) Veröffentlichungstag der Anmeldung :
**09.06.82 Patentblatt 82/23**

(45) Bekanntmachung des Hinweises auf die Patenterteilung : **25.07.84 Patentblatt 84/30**

(84) Benannte Vertragsstaaten :
**AT BE CH DE FR GB IT LI NL**

(56) Entgegenhaltungen :
**FR-A- 2 258 861**
**CHEMICAL ABSTRACTS, Band 89, Nr. 15, October 9, 1978, Seite 624, Zusammenfassung 129842t Columbus, Ohio (US) HIRANO, SHIGEHIRO et al.: "2-Deoxy-2-nicotinamido derivatives of some mono-, oligo-, and polysaccharides"**

(73) Patentinhaber : **F. HOFFMANN-LA ROCHE & CO.**
**Aktiengesellschaft**
**CH-4002 Basel (CH)**

(72) Erfinder : **Kiss, Joseph, Prof.**
**Hangstrasse 9**
**CH-4144 Arlesheim (CH)**

(74) Vertreter : **Mahé, Jean et al**
**Postfach 3255 Grenzacherstrasse 124**
**CH-4002 Basel (CH)**

## Beschreibung

Die vorliegende Erfindung betrifft ein neues Verfahren zur Herstellung eines β-D-Glucopyranosederivats, nämlich von 3,4,6-Tri-O-acetyl-2-desoxy-2-nicotinamido-1-O-nicotinoyl-β-D-glucopyranose.

Dieses Verfahren ist dadurch gekennzeichnet, dass man ein Säureadditionssalz des 2-Amino-2-desoxy-3,4,6-tri-O-acetyl-α-D-glucopyranosyl-bromids in Gegenwart einer Base mit Nicotinsäureanhydrid umsetzt.

Als Säureadditionssalze des 2-Amino-2-desoxy-3,4,6-tri-O-acetyl-α-D-glucopyranosyl-bromids kann man Mineralsäuresalze, wie das Hydrobromid, Hydrochlorid, Sulfat oder Nitrat, oder Salze mit starken organischen Säuren, wie das Oxalat oder Tartrat, vorzugsweise ein Mineralsäuresalz, insbesondere das Hydrobromid, verwenden.

Als Base kann ein tertiäres Amin, wie ein Tri-($C_{1-6}$-alkyl)-amin, z. B. Tri-(äthyl oder butyl)-amin, oder das N-Methylmorpholin oder 1,5-Diazabicyclo [5.4.0] undec-5-en (1,8-7), verwendet werden, wobei die Trialkylamine, insbesondere Triäthylamin, bevorzugt sind. Man kann auch eine anorganische Base, z. B. ein Alkali- oder Erdalkalimetall-hydroxyd, -hydrocarbonat oder -carbonat, wie Natrium- oder Kaliumcarbonat oder -hydrocarbonat, oder Calciumhydroxyd, verwenden.

Das erfindungsgemässe Verfahren wird zweckmässig in Gegenwart eines organischen Lösungsmittels, insbesondere eines Ketons, wie Aceton, Cyclohexanon oder Methyläthyl- oder Methylpropylketon ; eines Carbonsäureesters, wie Aethylacetat, eines Aethers, wie Dioxan oder Tetrahydrofuran ; oder Methylenchlorid, durchgeführt, wobei die Ketone, insbesondere Aceton, und die Carbonsäureester, insbesondere Aethylacetat, bevorzugt sind. Man kann aber auch in Abwesenheit von Lösungsmitteln arbeiten.

In einer bevorzugten Ausführungsform führt man das erfindungsgemässe Verfahren in Gegenwart eines tertiären oder quaternären Ammoniumnicotinats, wie eines Trialkyl- oder Tetraalkylammoniumnicotinats, z. B. des Tetrabutyl- oder vorzugsweise des Triäthylammoniumnicotinats, durch.

Vorzugsweise beträgt das Molverhältnis Nicotinsäure-anhydrid/Salz des 2-Amino-2-desoxy-3,4,6-tri-O-acetyl-α-D-glucopyranosyl-bromids mindestens 1/1 und das Molverhältnis Base/Salz des 2-Amino-2-desoxy-3,4,6-tri-O-acetyl-α-D-glucopyranosyl-bromids mindestens 2/1.

Die Temperatur, bei der das erfindungsgemässe Verfahren durchgeführt wird, ist nicht kritisch. Zweckmässig wird zwischen 0 und 50 °C, insbesondere bei Raumtemperatur, gearbeitet.

Die Säureadditionssalze des 2-Amino-2-desoxy-3,4,6-tri-O-acetyl-α-D-glucopyranosyl-bromids können in an sich bekannter Weise, z. B. in Analogie zu der in J. Chem. Soc. 99 (1911), 250 beschriebenen Methode zur Herstellung von 2-Amino-2-desoxy-3,4,6-tri-O-acetyl-α-D-glucopyranosyl-bromid-hydrobromid durch Umsetzung von D-Glucosamin-hydrochlorid mit Acetylbromid, hergestellt werden.

Beispiel 1

100 g (0,222 Mol) 2-Amino-2-desoxy-3,4,6-tri-O-acetyl-α-D-glucopyranosyl-bromid-hydrobromid und 120 g (0,525 Mol) Nicotinsäureanhydrid werden in 2 500 ml Aceton aufgeschlämmt und im Eisbad auf + 10° abgekühlt. Zu dem Reaktionsgemisch werden unter Rühren 150 ml (118,3 g, 1,17 Mol) Triäthylamin innert 5 Minuten gegeben. Das Reaktionsgemisch wird bei Raumtemperatur für 15-18 Stunden gerührt. Festes Material wird abgenutscht. Der Nutschkuchen wird mit 250 ml Aceton gewaschen und die vereinigten Acetonlösungen werden im Vakuum bei etwa 35-40° eingedampft. Der Rückstand wird in 500 ml Methylenchlorid aufgenommen, mit 1 000 ml Wasser gewaschen und über 100 g Natriumsulfat getrocknet. Die abgenutschte Lösung wird bei 35-40° eingedampft, der erhaltene Rückstand (130 g) in 100 ml Aethylacetat aufgelöst. Die kalte Lösung wird mit Kristallen von 3,4,6-Tri-O-acetyl-2-desoxy-2-nicotinamido-1-O-nicotinoyl-β-D-glucopyranose angeimpft und für einige Stunden im Kühlschrank stehengelassen. Die ausgeschiedenen Kristalle werden abgenutscht, mit 2 × 50 ml eines Aethylacetats (60 %)-n-Hexan (40 %)-Gemisches gewaschen. Man erhält 46-52 g leicht graugefärbtes, kristallines 3,4,6-Tri-O-acetyl-2-desoxy-2-nicotinamido-1-O-nicotinoyl-β-D-glucopyranose, Smp. 174-176°. Aus der Mutterlauge werden noch 3-4 g Material vom Schmelzpunkt 172-174° erhalten. Ausbeute : 43-45 %.

Das Rohprodukt wird aus 250 ml Aethylacetat heiss umkristallisiert. 42-44 g weisses, kristallines Produkt vom Schmelzpunkt 185-186° werden erhalten.

Das Ausgangsmaterial kann wie folgt hergestellt werden :

100 g (0,475 Mol) D-Glucosamin-hydrochlorid werden in 250 ml (416 g, 3,39 Mol) Acetylbromid aufgeschlämmt und bei Raumtemperatur gerührt. Das Reaktionsgemisch wird nach 24-stündiger Reaktionszeit in 250 ml Chloroform aufgeschlämmt und im Vakuum zur Trockene eingedampft. Der feste Rückstand wird in 1 500 ml Chloroform aufgelöst. Unlösliches Glucosamin-hydrochlorid wird abgenutscht und die Lösung wird im Vakuum auf die Hälfte eingeengt. Zur warmen Lösung werden 700 ml Diäthyläther gegeben. Die ausgeschiedenen Kristalle werden nach 2-stündigem Stehen im Kühlschrank abgenutscht. Die Kristalle werden mit 100 ml Diäthyläther gewaschen und im Vakuum bei 50° 2 Stunden getrocknet. 178,2 g 2-Amino-2-desoxy-3,4,6-tri-O-acetyl-α-D-glucopyranosyl-bromidhydrobromid werden erhalten (85,5 %), Smp. 156-158°.

Beispiel 2

Zu einem Gemisch von 20 g 2-Amino-2-desoxy-3,4,6-tri-O-acetyl-α-D-glucopyranosyl-bromid-hydrobromid, 12 g Nicotinsäureanhydrid und 12 g Triäthylammoniumnicotinat in 400 ml Aethylacetat werden unter Rühren bei 3-5° 40 ml Triäthylamin gegeben. Dann wird festes Material bei Raumtemperatur abgenutscht. Der Nutschkuchen wird mit Aethylacetat gewaschen und die vereinigten Waschlösungen werden eingedampft. Der ölige Rückstand wird in 300 ml Methylenchlorid aufgenommen und mit Eiswasser extrahiert. Die wässerigen Phasen werden mit Methylenchlorid extrahiert. Die vereinigten Methylenchloridphasen werden getrocknet und eingedampft. Der Rückstand wird in 40 ml eines Aethylacetat (60 %)-n-Hexan (40 %)-Gemisches aufgenommen. Die ausfallenden Kristalle werden abgenutscht und mit dem gleichen Lösungsmittelgemisch gewaschen. Man erhält 13,5 g (59 %) 3,4,6-Tri-O-acetyl-2-desoxy-2-nicotinamido-1-O-nicotinoyl-β-D-glucopyranose, Smp. 175-177°.

Beispiel 3

Zu einem Gemisch von 20 g 2-Amino-2-desoxy-3,4,6-tri-O-acetyl-α-D-glucopyranosyl-bromid-hydrobromid und 24 g Nicotinsäureanhydrid in 400 ml Aceton werden unter Rühren bei 3-5° 40 ml Triäthylamin gegeben. Nach Aufarbeitung des Reaktionsgemisches, wie im Beispiel 2, wird das gleiche Produkt in etwa gleicher Ausbeute erhalten.

Beispiel 4

Zu einem Gemisch von 20 g 2-Amino-2-desoxy-3,4,6-tri-O-acetyl-α-D-glucopyranosyl-bromid-hydrobromid und 24 g Nicotinsäureanhydrid werden unter Rühren bei Raumtemperatur 400 ml Triäthylamin gegeben. Nach Aufarbeitung des Reaktionsgemisches, wie im Beispiel 2, wird das gleiche Produkt in etwa gleicher Ausbeute erhalten.

**Ansprüche**

1. Verfahren zur Herstellung von 3,4,6-Tri-O-acetyl-2-desoxy-2-nicotinamido-1-O-nicotinoyl-β-D-glucopyranose dadurch gekennzeichnet, dass man ein Säureadditionssalz des 2-Amino-2-desoxy-3,4,6-tri-O-acetyl-α-D-glucopyranosyl-bromids in Gegenwart einer Base mit Nicotinsäureanhydrid umsetzt.

2. Verfahren nach Anspruch 1, dadurch gekennzeichnet, dass man als Säureadditionssalz des 2-Amino-2-desoxy-3,4,6-tri-O-acetyl-α-D-glucopyranosyl-bromids ein Mineralsäuresalz, insbesondere das Hydrobromid, verwendet.

3. Verfahren nach Anspruch 1 oder 2, dadurch gekennzeichnet, dass man als Base ein tertiäres Amin, insbesondere ein Tri-(C$_{1-6}$-alkyl)-amin, vorzugsweise Triäthylamin, verwendet.

4. Verfahren nach einem der Ansprüche 1-3, dadurch gekennzeichnet, dass man die Umsetzung in einem organischen Lösungsmittel, insbesondere einem Keton, vorzugsweise Aceton, oder einem Carbonsäureester, vorzugsweise Aethylacetat, durchführt.

5. Verfahren nach einem der Ansprüche 1-4, dadurch gekennzeichnet, dass man die Umsetzung in Gegenwart eines tertiären oder quaternären Ammoniumnicotinats, insbesondere eines Trialkyl- oder Tetraalkylammoniumnicotinats, vorzugsweise des Triäthylammoniumnicotinats, durchführt.

**Claims**

1. A process for the manufacture of 3,4,6-tri-O-acetyl-2-desoxy-2-nicotinamido-1-O-nicotinoyl-β-D-glucopyranose, characterized by reacting an acid addition salt of 2-amino-2-desoxy-3,4,6-tri-O-acetyl-α-D-glucopyranosyl bromide with nicotinic acid anhydride in the presence of a base.

2. A process according to claim 1, characterized in that a mineral acid salt, especially the hydrobromide, is used as the acid addition salt of 2-amino-2-desoxy-3,4,6-tri-O-acetyl-α-D-glucopyranosyl bromide.

3. A process according to claim 1 or 2, characterized in that a tertiary amine, especially a tri-(C$_{1-6}$-alkyl)-amine, preferably triethylamine, is used as the base.

4. A process according to any one of claims 1-3, characterized in that the reaction is carried out in an organic solvent, especially a ketone, preferably acetone, or a carboxylic acid ester, preferably ethyl acetate.

5. A process according to any one of claims 1-4, characterized in that the reaction is carried out in the presence of a tertiary or quaternary ammonium nicotinate, especially a trialkylammonium nicotinate or tetraalkyl-ammonium nicotinate, preferably triethylammonium nicotinate.

**Revendications**

1. Procédé de préparation du 3,4,6-tri-O-acétyl-2-désoxy-2-nicotinamido-1-O-nicotinoyl-bêta-D-glucopyrannose, caractérisé en ce que l'on fait réagir un sel, formé par addition avec un acide, du bromure de 2-amino-2-désoxy-3,4,6-tri-O-acétyl-alpha-D-glucopyrannosyle, en présence d'une base, avec l'anhydride nicotinique.

2. Procédé selon la revendication 1, caractérisé en ce que le sel du bromure de 2-amino-2-désoxy-3,4,6-tri-O-acétyl-alpha-D-glucopyrannosyle formé par addition avec un acide est un sel d'acide minéral, en particulier le bromhydrate.

3. Procédé selon la revendication 1 ou 2, caractérisé en ce que l'on utilise en tant que base une amine tertiaire, en particulier une tri-(alkyle en $C_1$-$C_6$)-amine, de préférence la triéthylamine.

4. Procédé selon l'une des revendications 1 à 3, caractérisé en ce que l'on effectue la réaction dans un solvant organique, en particulier une cétone, de préférence l'acétone, ou un ester d'acide carboxylique, de préférence l'acétate d'éthyle.

5. Procédé selon l'une des revendications 1 à 4, caractérisé en ce que l'on effectue la réaction en présence d'un nicotinate d'ammonium tertiaire ou quaternaire, en particulier un nicotinate de trialkyl- ou tétraalkyl-ammonium, de préférence le nicotinate de triéthylammonium.